(19) Europäisches Patentamt
European Patent Office
Office européen des brevets

(11) Veröffentlichungsnummer: **0 004 030**
**B1**

(12) # EUROPÄISCHE PATENTSCHRIFT

(45) Veröffentlichungstag der Patentschrift: **30.12.81**

(51) Int. Cl.³: **C 07 C 127/22**, A 01 N 47/34

(21) Anmeldenummer: **79100599.4**

(22) Anmeldetag: **01.03.79**

(54) **Substituierte N-Phenyl-N'-fluorbenzoylharnstoffe, Verfahren zu ihrer Herstellung und ihre Verwendung in der Schädlingsbekämpfung.**

(30) Priorität: **13.03.78 CH 2700/78**
**30.01.79 CH 884/79**

(43) Veröffentlichungstag der Anmeldung: **19.09.79 Patentblatt 79/19**

(45) Bekanntmachung des Hinweises auf die Patenterteilung: **30.12.81 Patentblatt 81/52**

(84) Benannte Vertragsstaaten: **BE CH DE FR GB IT NL**

(56) Entgegenhaltungen:
**DE-A-2 123 236**

(73) Patentinhaber: **CIBA-GEIGY AG, Patentabteilung Postfach, CH-4002 Basel (CH)**

(72) Erfinder: **Ehrenfreund, Josef, Dr., Langenhagweg 11, CH-4123 Allschwil (CH)**

**EP 0 004 030 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Substituierte N-Phenyl-N'-fluorbenzoylharnstoffe, Verfahren zu ihrer Herstellung und ihre Verwendung in der Schädlingsbekämpfung

Die vorliegende Erfindung betrifft neue substituierte N-Phenyl-N'-fluorbenzoylharnstoffe, Verfahren zu ihrer Herstellung und ihre Verwendung in der Schädlingsbekämpfung.

Die erfindungsgemässen substituierten N-Phenyl-N'-fluorbenzoylharnstoffe haben die Formel I

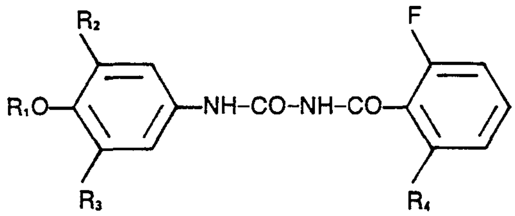 (I)

worin

$R_1$ einen $C_2$-$C_3$-Alkenylrest, einen ein- oder zweifach mit Chlor substituierten $C_2$-$C_4$-Alkenylrest oder den Propargylrest;

$R_2$ und $R_3$ unabhängig voneinander Wasserstoff oder Chlor; und

$R_4$ Wasserstoff oder Fluor bedeuten.

Wegen ihrer Wirkung als Schädlingsbekämpfungsmittel bevorzugt sind erfindungsgemässe Verbindungen der Formel I, die dadurch gekennzeichnet sind, dass $R_2$ und $R_3$ Chlor bedeuten. Hervorzuheben sind daneben solche Verbindungen der Formel I, worin $R_1$ einen einfach mit Chlor substituierten $C_2$-$C_4$-Alkenylrest, insbesondere den Rest $Cl-CH=CH-CH_2-$ darstellt. Wertvoll sind aufgrund ihrer biologischen Wirksamkeit ferner die Verbindungen der Formel I, worin $R_1$ für den Rest

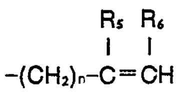

steht, wobei $R_5$ und $R_6$ unabhängig voneinander Wasserstoff oder Chlor bedeuten.

Von besonderem Interesse sind auch Verbindungen der Formel I, worin $R_2$ und $R_3$ Wasserstoff bedeuten. Besonders wirksam sind die erfindungsgemässen Verbindungen der Formel I, bei denen $R_4$ Fluor darstellt.

Die Verbindungen der Formel I fallen — wo dies prinzipiell möglich ist — als cis/trans-Isomerengemische an. In diesem Sinne sind als Verbindungen der Formel I sowohl die cis- oder trans-Formen als auch die entsprechenden Isomerengemische zu verstehen. Ein Isomerengemisch kann z.B. mit Hilfe der bekannten chromatographischen Trennmethoden und anschliessenden Eluierung in die isomeren Formen getrennt werden. Zur Synthese stereochemisch einheitlicher Verbindungen der Formel I verwendet man mit Vorteil stereochemisch einheitliche Ausgangsverbindungen der nachstehenden Formeln II oder IV.

Die Verbindungen der Formel I können nach an sich bekannten Verfahren hergestellt werden (vgl. u.a. die deutschen Offenlegungsschriften Nr. 2 123 236, 2 601 780).

So kann man z.B. eine Verbindung der Formel I erhalten durch Umsetzung

a) einer Verbindung der Formel II

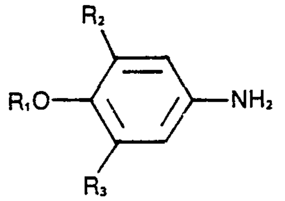 (II)

mit einer Verbindung der Formel III

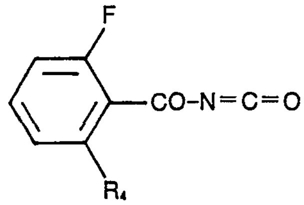 (III)

oder

b) einer Verbindung der Formel IV

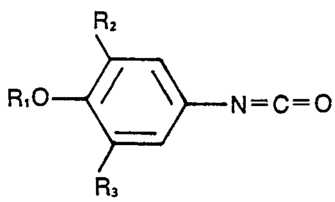 (IV)

mit einer Verbindung der Formel V

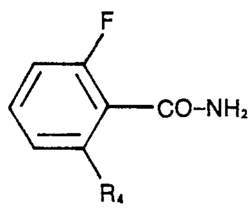 (V)

In den obigen Formeln II, III, IV und V haben die Reste $R_1$, $R_2$, $R_3$ und $R_4$ die unter Formel I vorstehend angegebenen Bedeutungen.

Die erwähnten Verfahren a) und b) können vorzugsweise unter normalem Druck und in Gegenwart eines organischen Lösungs- oder Verdünnungsmittels durchgeführt werden. Als Lösungs- oder Verdünnungsmittel eignen sich z.B. Äther und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von −10 bis 100°C, vorzugsweise zwischen 15 und 25°C, gegebenenfalls in Gegenwart einer organischen Base, z.B. Triäthylamin, durchgeführt. Die Durchführung von Verfahren b) erfolgt bei einer Temperatur von 0 bis 120°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium.

Die Ausgangsstoffe der Formeln II, III, IV und V sind bekannt oder können analog bekannten Verfahren hergestellt werden.

Es ist bereits bekannt, dass bestimmte N-Phenyl-N'-benzoylharnstoffe insektizide Eigenschaften besitzen (vgl. deutsche Offenlegungsschriften 2 123 236, 2 504 982, 2 537 413 und 2 601 780, die belgischen Patentschriften 832 304, 843 906, 844 066 und 867 046 sowie die US-Patentschrift 4 089 975).

Überraschenderweise wurde nun gefunden, dass die erfindungsgemässen N-Phenyl-N'-fluorbenzoylharnstoffe der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Planzen und Tiere befallenden Schädlingen.

Insbesondere eignen sich die Verbindungen der Formel I zur Bekämpfung von Insekten der Familien: Acrididae, Blattidae, Gryllidae, Gryllotalpidae, Tettigoniidae, Cimicidae. Phyrrhocoridae, Reduviidae, Aphididae, Delphacidae, Diaspididae, Pseudococcidae, Chrysomelidae, Coccinellidae, Bruchidae, Scarabaeidae, Dermestidae, Tenebrionidae, Curculionidae, Tineidae, Noctuidae, Lymantriidae, Pyralidae, Galleridae, Culicidae, Tipulidae, Stomoxydae, Muscidae, Calliphoridae, Trypetidae, Pulicidae sowie zur Bekämpfung von Akariden der Familien: Ixodidae, Argasidae, Tetranychidae, Dermanyssidae.

Neben ihrer sehr günstigen Wirkung gegenüber Fliegen, wie z.B. Musca domestica, und Mückenlarven eignen sich Verbindungen der Formel I auch zur Bekämpfung von pflanzenschädigenden Frassinsekten, in Zier- und Nutzpflanzen, insbesondere in Baumwollkulturen (z.B. gegen Spodoptera littoralis und Heliothis virescens) sowie in Gemüsekulturen (z.B. gegen Leptinotarsa decemlineata und Pieris brassicae). Hervorzuheben ist ferner die ovizide bzw. ovolarvizide Wirkung von Verbindungen der Formel I.

Die Verbindungen der Formel I eignen sich weiterhin zur Bekämpfung von Ektoparasiten an Haus- und Nutztieren, z.B., durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht:
organische Phosphorverbindungen,
Nitrophenole und Derivate,
Formamidine, Harnstoffe,
Carbamate und
chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,-9-trioxaundecan oder S,S,S-Tributylphosphorotrithioate.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind «cattle dips», d.h. Viehbäder, und «spray races», d.h. Sprühgänge, in denen wässrige Zubereitungen verwendet werden, zu erwähnen. Diese Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I mit den geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;

b) Lösungen.

Der Gehalt an Wirkstoff in den oben beschriebenen Mitteln liegt zwischen 0,1 bis 95%.

Wie Wirkstoffe der Formel I können beispielsweise wie folgt formuliert werden:

Stäubemittel: Zur Herstellung eines a) 5%igen und b) 2%igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit den Trägerstoffen vermischt und vermahlen.

Granulat: Zur Herstellung eines 5%igen Granulates werden die folgenden Stoffe verwendet:

5 Teile Wirkstoff,
0,25 Teile Epichlorhydrin,
0,25 Teile Cetylpolyglykoläther,
3,50 Teile Polyäthylenglykol,
91 Teile Kaolin (Korngrösse 0,3-0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen, d) 10%igen Spritzpulvers werden folgende Bestandteile verwendet:

a) 40 Teile Wirkstoff,
5 Teile Ligninsulfonsäure-Natriumsalz,
1 Teil Dibutylnaphthalinsulfonsäure-Natriumsalz,
54 Teile Kieselsäure;

b) 25 Teile Wirkstoff,
4,5 Teile Calcium-Ligninsulfonat,
1,9 Teile Champagne-Kreide/Hydroxyäthylcellulose-Gemisch (1 : 1),
1,5 Teile Natrium-dibutyl-naphthalinsulfonat,
19,5 Teile Kieselsäure,
19,5 Teile Champagne-Kreide,
28,1 Teile Kaolin;

c) 25 Teile Wirkstoff,
2,5 Teile Isooctylphenoxy-polyoxyäthylenäthanol,
1,7 Teile Champagne-Kreide/Hydroxyäthylcellulose-Gemisch (1 : 1),
8,3 Teile Natriumaluminiumsilikat,
16,5 Teile Kieselgur,
46 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate: Zur Herstellung eines a) 10%igen, b) 25%igen und c) 50%igen emulgierbaren Konzentrates werden folgende Stoffe verwendet:

a) 10 Teile Wirkstoff,
3,4 Teile epoxydiertes Pflanzenöl,
3,4 Teile eines Kombinationsemulgators, bestehend aus Fettalkoholpolyglykoläther und Alkylaralkyl-sulfonat-Calcium-Salz,
40 Teile Dimethylformamid,
43,2 Teile Xylol;

b) 25 Teile Wirkstoff,
2,5 Teile epoxydiertes Pflanzenöl,
10 Teile eines Alkylarylsulfonat/Fettalkoholpolyglykoläther-Gemisches,
5 Teile Dimethylformamid,
57,5 Teile Xylol;

c) 50 Teile Wirkstoff,
4,2 Teile Tributylphenol-Polyglykoläther,
5,8 Teile Calcium-Dodecylbenzolsulfonat,
20 Teile Cyclohexanon,
20 Teile Xylol.

Aus solchen Konzentrationen können durch Verdünnen mit Wasser Emulsionen jeder gewünschten Konzentration hergestellt werden.

Sprühmittel: Zur Herstellung eines a) 5%igen und b) 95%igen Sprühmittels werden die folgenden Bestandteile verwendet:

a) 5 Teile Wirkstoff,
1 Teil Epichlorhydrin,
94 Teile Benzin (Siedegrenzen 160-190°C);

b) 95 Teile Wirkstoff,
5 Teile Epichlorhydrin.

Beispiel 1

Zu einer Lösung von 3,3 g N-3,5-Dichlor-4-(3'-chlorallyloxy)-anilin in 30 ml absolutem Äther werden bei Raumtemperatur 3 g 2,6-Difluorbenzoylisocyanat, gelöst in 10 ml absolutem Äther, zugetropft. Der nach kurzer Zeit ausfallende Niederschlag wird abgesaugt und mit Äther gewaschen. Durch Umkristallisation aus Essigsäureäthylester erhält man analysenreinen N-3,5-Dichlor-4-(3'-chlorallyloxy)-phenyl-N'-difluorbenzoylharnstoff vom Schmelzpunkt 135-137°C (als Gemisch der Stereoisomeren).

Beispiel 2

Ein Gemisch aus 5,0 g N-3,5-Dichlor-4-allyloxy-phenylisocyanat, 3,5 g 2,6-Difluorbenzamid, 0,5 g Natrium und 25 ml Pyridin wird während 24 Stunden auf 100°C erhitzt. Danach giesst man das Reaktionsgemisch auf Eis und wäscht den ausgefallenen Feststoff mit Wasser und Alkohol. Anschliessend wird der Niederschlag aus Acetonitril umkristallisiert. Man erhält so N-3,5-Dichlor-4-allyloxyphenyl-N'-2,6-difluorbenzoylharnstoff vom Schmelzpunkt 161-163°C.

Analog den in den vorstehenden Beispielen beschriebeneen Arbeitsweisen werden die folgenden Verbindungen der Formel I hergestellt:

| $R_1$ | $R_2$ | $R_3$ | $R_4$ | Schmelzpunkt [°C] |
|---|---|---|---|---|
| $CH_2=CCl-CH_2-$ | Cl | Cl | F | 172-174 |
| $Cl_2C=CH-CH_2-$ | Cl | Cl | F | 176-178 |
| $HC\equiv C-CH_2-$ | Cl | Cl | F | 226-227 |
| $ClCH=CCl-$ | Cl | Cl | F | 190-192 |
| $ClCH=CCl-CH_2-$ | Cl | Cl | F | 188-193 *) |
| $ClCH=CH-CH_2-$ | H | H | F | 162-180 *) |
| $ClCH=CCl-$ | H | H | F | 178-179 *) |
| $CH_2=CH-CH_2-$ | Cl | Cl | H | 165-166 |
| $CH_2=CCl-CH_2-$ | Cl | Cl | H | 190-192 |
| $ClCH=CH-CH_2-$ | Cl | Cl | H | 177-178 *) |
| $ClCH=CCl-$ | Cl | Cl | H | 171-172 |
| $Cl_2C=CH-CH_2-$ | Cl | H | F | |
| $ClCH=CH-CH_2-$ | Cl | H | F | 164-186 *) |
| $CH_2=CCl-CH_2-$ | Cl | H | F | 180-181 |
| $CH_2=CH-CH_2$ | Cl | H | H | 182-184 |
| $CH_2=CH-CH_2-$ | Cl | H | F | 182-184 |
| $CH_2=CH-CH_2-$ | H | H | F | 174-176 |
| $ClCH=CH-CH_2$ | Cl | H | H | 143-147 *) |
| $CH_3-CCl-CH-CH_2$ | Cl | Cl | F | 156-158 |
| $CH_3-CCl=CH-CH_2$ | H | H | F | 187-188 |
| $CH_2=CCl-CH_2$ | Cl | H | H | 159-160 |
| $ClCH=CCl-CH_2-$ | Cl | H | H | *) |
| $ClCH=CCl-CH_2-$ | Cl | H | F | *) |
| $Cl_2C=CH-CH_2-$ | Cl | H | H | |
| $Cl_2C=CH-$ | Cl | H | H | |
| $Cl_2C=CH-$ | Cl | H | F | |
| $Cl_2C=CH-$ | H | H | H | |
| $Cl_2C=CH-$ | H | H | F | |
| $ClCH=CCl-$ | Cl | H | F | 197-203 |
| $ClCH=CCl-$ | Cl | H | H | 182-183 |
| $CH_3-CCl=CH-CH_2-$ | Cl | Cl | H | 182-183 *) |

*) Stereoisomerengemisch

Beispiel 3

Zu einer Lösung von 2,5 g 3,5-Dichlor-4-(3'-trans-chlorallyloxy)-anilin in 30 ml absolutem Äther werden bei Raumtemperatur 1,85 g 2,6-Difluorbenzoylisocyanat, gelöst in 10 ml absolutem Äther, zugetropft. Der nach kurzer Zeit ausfallende Niederschlag wird abgesaugt und mit Äther gewaschen. Durch Umkristallisation aus Toluol erhält man isomerenreinen N-3,5-Dichlor-4-(3'-trans-chlorallyloxy)-phenyl-N'-2,6-difluorbenzoylharnstoff vom Schmelzpunkt 175,5 bis 177,5°C.

Auf analoge Weise, d.h. ausgehend von den entsprechenden stereochemisch einheitlichen Anilinen, erhält man die folgenden Verbindungen:

| $R_1$ | $R_2$ | $R_3$ | $R_4$ | Schmelzpunkt [°C] |
|---|---|---|---|---|
| $ClCH=CH-CH_2-$ | Cl | Cl | F | 145-146,5 (cis-Isomer) |
| $ClCH=CH-CH_2-$ | H | H | F | 196-198 (trans-Isomer) |
| $ClCH=CH-CH_2$ | H | H | F | 170-172 (cis-Isomer) |

Beispiel 4

Wirkung gegen Musca domestica:

Je 50 frisch zubereites CSMA-Nährsubstrat für Maden wurde in Becher eingewogen. Von einer 1 gew.%igen acetonischen Lösung des betreffenden Wirkstoffes wurde eine bestimmte Menge auf das in den Bechern befindliche Nährsubstrat pipettiert. Nach dem Durchmischen des Substrates lässt man das Aceton mindestens 20 Stunden lang verdampfen.

Dann wurden pro Wirkstoff und Konzentration je 25 eintägige Maden von Musca domestica

in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen bestimmt.

Verbindungen gemäss den Beispielen 1 bis 3 zeigten gute Wirkung im obigen Test.

Beispiel 5
Wirkung gegen Lucilia sericata:

Zu 9 ml eines Zuchtmediums wurden bei 50°C 1 ml einer 0,5% Aktivsubstanz enthaltenden wässrigen Lösung gegeben. Nun wurden ca. 30 frisch geschlüpfte Lucilia sericata-Larven zum Zuchtmedium gegeben und nach 48 und 96 Stunden die insektizide Wirkung durch Ermittlung der Abtötungsrate festgestellt.

Verbindungen gemäss den Beispielen 1 bis 3 zeigten in diesem Test gute Wirkung gegen Lucilia sericata.

Beispiel 6
Wirkung gegen Aëdes aegypti:

Auf die Oberfläche von 150 ml Wasser, das sich in einem Behälter befindet, wurde so viel einer 0,1%igen acetonischen Lösung des Wirkstoffes pipettiert, dass Konzentrationen von je 10, 5 und 1 ppm erhalten wurden. Nach Verdunsten des Acetons wurde der Behälter mit 30-40 2tägigen Aëdes-Larven beschickt. Nach 1, 2 und 5 Tagen wurde die Mortalität geprüft.

Verbindungen gemäss den Beispielen 1 bis 3 zeigten in diesem Test gute Wirkung gegen Aëdes aegypti.

Beispiel 7
Insektizide Frassgift-Wirkung:

Baumwollpflanzen wurden mit einer 0,05%igen wässrigen Wirkstoffemulsion (erhalten aus einem 10%igen emulgierbaren Konzentrat) besprüht.

Nach dem Antrocknen des Belages wurden die Baumwollpflanzen je mit Spodoptera littoralis- und Heliothis virescens-Larven im dritten larvalen Stadium besetzt. Der Versuch wurde bei 24°C und 60% relativer Luftfeuchtigkeit durchgeführt.

Verbindungen gemäss den Beispielen 1 bis 3 zeigten im obigen Test gute insektizide Frassgift-Wirkung gegen Spodoptera- und Heliothis-Larven.

Beispiel 8
Ovizide Wirkung auf Spodoptera littoralis:

Auf Filterpapier abgelegte Eier von Spodoptera littoralis wurden aus dem Papier ausgeschnitten und in eine 0,05 gew.%ige Lösung des Wirkstoffes in einem Aceton-Wasser-Gemisch (1 : 1) getaucht. Die so behandelten Eiablagen wurden dann aus diesem Gemisch herausgenommen und bei 21°C und 60% relativer Feuchtigkeit in Kunststoffschafen deponiert.

Nach 3 bis 4 Tagen wurde die Schlupfrate, d.h. die Anzahl der Larven, die sich aus den behandelten Eiern entwickelt hatten, bestimmt.

Die erfindungsgemässen Verbindungen gemäss den Beispielen 1-3 zeigten gute Wirkung in obigem Test.

**Patentansprüche**

1. Verbindung der Formel I

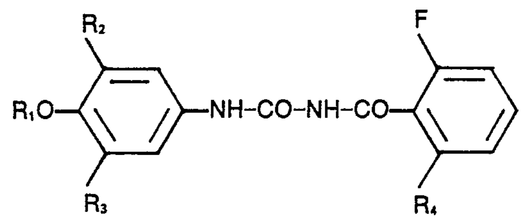

(I)

worin

$R_1$ einen $C_2$-$C_3$-Alkenylrest, einen ein- oder zweifach mit Chlor substituierten $C_2$-$C_4$-Alkenylrest oder den Propargylrest:

$R_2$ und $R_3$ unabhängig voneinander Wasserstoff oder Chlor; und

$R_4$ Wasserstoff oder Fluor bedeuten.

2. Verbindung der Formel I gemäss Anspruch 1, dadurch gekennzeichnet, dass $R_2$ und $R_3$ Chlor bedeuten.

3. Verbindung der Formel I gemäss Anspruch 1 oder 2, dadurch gekennzeichnet, dass $R_1$ einen einfach mit Chlor substituierten $C_2$-$C_4$-Alkenylrest bedeutet.

4. Verbindung der Formel I gemäss Anspruch 3, dadurch gekennzeichnet, dass $R_1$ den Rest $Cl-CH=CH-CH_2-$ bedeutet.

5. Verbindung der Formel I gemäss Anspruch 1 oder 2, dadurch gekennzeichnet, dass $R_1$ den Rest

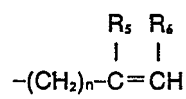

darstellt, wobei $R_5$ und $R_6$ unabhängig voneinander Wasserstoff oder Chlor und n Null oder 1 bedeuten.

6. Verbindung der Formel I gemäss Anspruch 1, 3, 4 oder 5, dadurch gekennzeichnet, dass $R_2$ und $R_3$ Wasserstoff bedeuten.

7. Verbindung der Formel I gemäss Anspruch 1 bis 6, dadurch gekennzeichnet, dass $R_4$ Fluor bedeutet.

8. Verbindung gemäss Anspruch 7 der Formel

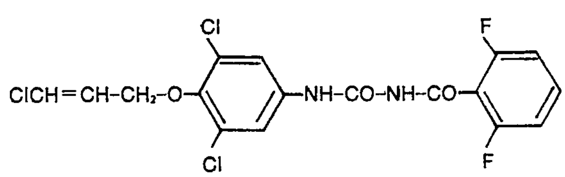

9. Verbindung gemäss Anspruch 7 der Formel

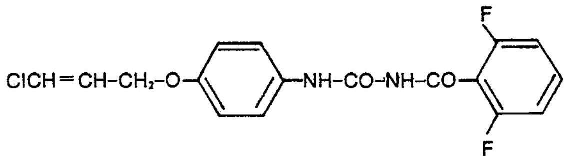

10. Verbindung gemäss Anspruch 7 der Formel

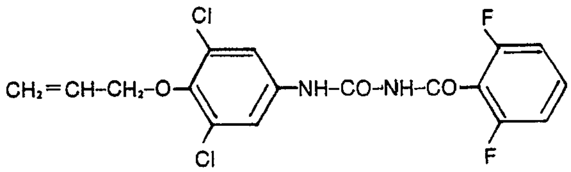

11. Verbindung gemäss Anspruch 2 der Formel

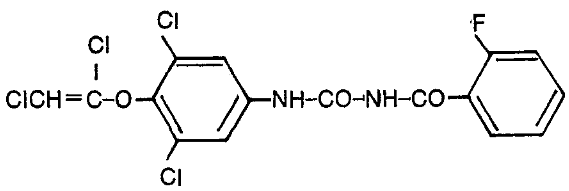

12. Verbindung gemäss Anspruch 2 der Formel

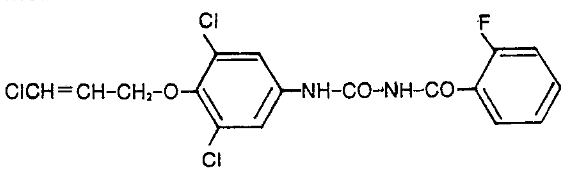

13. Verfahren zur Herstellung einer Verbindung gemäss den Ansprüchen 1 bis 12, dadurch gekennzeichnet, dass man

a) eine Verbindung der Formel II

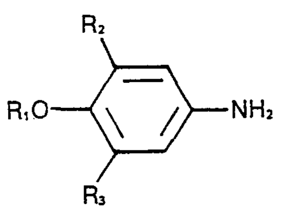

(II)

mit einer Verbindung der Formel III

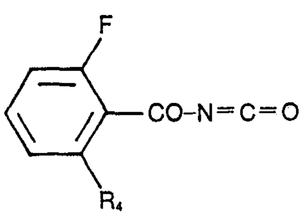

(III)

oder

b) eine Verbindung der Formel IV

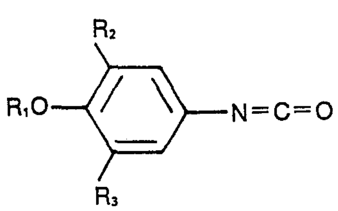

(IV)

mit einer Verbindung der Formel V

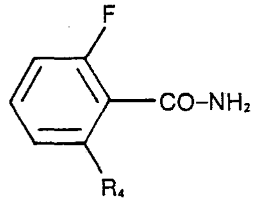

(V)

umsetzt, wobei in den Formeln II bis V die Reste $R_1$, $R_2$, $R_3$ und $R_4$ die in den Ansprüchen 1 bis 7 angegebenen Bedeutungen haben.

14. Schädlingsbekämpfungsmittel, dadurch gekennzeichnet, dass es als aktive Komponente eine Verbindung gemäss den Ansprüchen 1 bis 12 zusammen mit geeigneten Trägern und/oder anderen Zuschlagstoffen enthält.

15. Verwendung einer Verbindung gemäss den Ansprüchen 1 bis 12 zur Bekämpfung von Schädlingen, vorzugsweise von Insekten und Vertretern der Ordnung Akarina.

**Claims**

1. A compound of the formula I

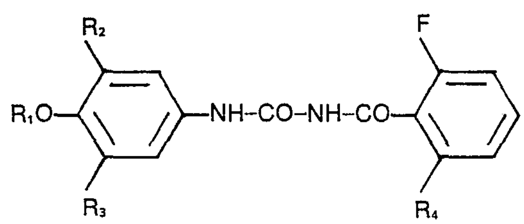

(I)

in which

$R_1$ is a $C_2$-$C_3$-alkenyl group, a $C_2$-$C_4$-alkenyl group mono- or disubstituted by chlorine, or it is the propargyl group,

$R_2$ and $R_3$ independently of one another are hydrogen or chlorine, and

$R_4$ is hydrogen or fluorine.

2. A compound of the formula I according to Claim 1 wherein $R_2$ and $R_3$ are each chlorine.

3. A compound of the formula I according to Claim 1 or 2, wherein $R_1$ is a $C_2$-$C_4$-alkenyl group which is monosubstituted by chlorine.

4. A compound of the formula I according to Claim 3 wherein $R_1$ is the radical $Cl-CH=CH-CH_2-$.

5. A compound of the formula I according to Claim 1 or 2, wherein $R_1$ is the radical

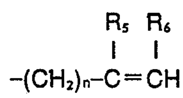

in which $R_5$ and $R_6$ independently of one another are each hydrogen or chlorine, and n is naught or 1.

6. A compound of the formula I according to Claim 1, 3, 4 or 5, wherein $R_2$ and $R_3$ are each hydrogen.

7. A compound of the formula I according to Claims 1 to 6, wherein $R_4$ is fluorine.

8. A compound according to Claim 7 of the formula

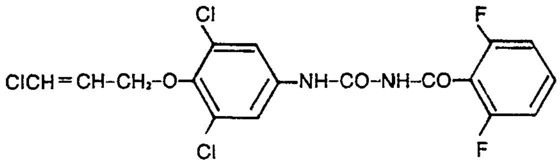

9. A compound according to Claim 7 of the formula

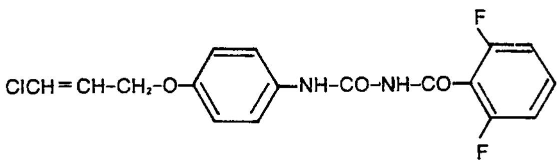

10. A compound according to Claim 7 of the formula

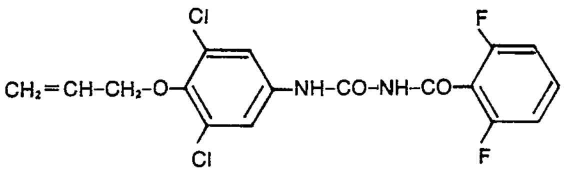

11. A compound according to Claim 2 of the formula

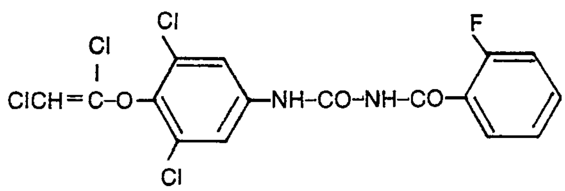

12. A compound according to Claim 2 of the formula

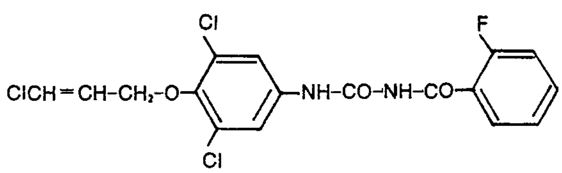

13. A process for producing a compound according to Claims 1 to 12, which process comprises reacting
a) a compound of the formula II

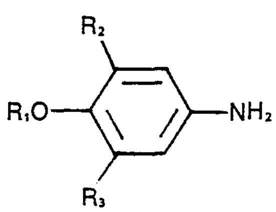

(II)

with a compound of the formula III

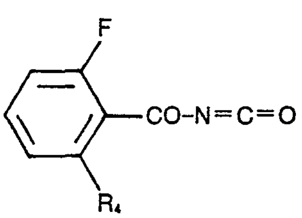

(III)

or
b) a compound of the formula IV

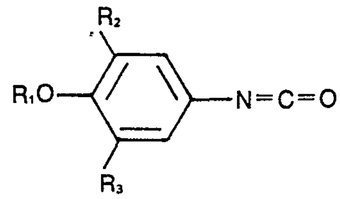

(IV)

with a compound of the formula V

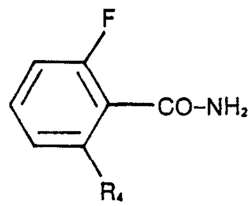

(V)

where in the formulae II to V, the radicals $R_1$, $R_2$, $R_3$ and $R_4$ have the meanings given in Claims 1 to 7.

14. A pesticidal composition containing a compound according to Claims 1 to 12 as active ingredient, together with suitable carriers and/or other additives.

15. Use of a compound according to Claims 1 to 12 for combating pests, preferably insects, and representatives of the order Acarina.

**Revendications**

1. Composé de formule

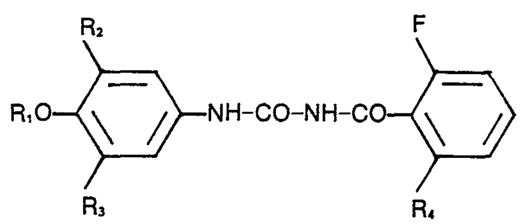

(I)

dans laquelle
$R_1$ représente un reste alcényle en $C_2$-$C_3$, un reste alcényle en $C_2$-$C_4$ mono- ou di-substitué par le chlore, ou le reste propargyle;
$R_2$ et $R_3$ représentent chacun, indépendamment l'un de l'autre, l'hydrogène ou le chlore et
$R_4$ représente l'hydrogène ou le fluor.

2. Composé de formule I selon la revendication 1, caractérisé en ce que $R_2$ et $R_3$ représentent le chlore.

3. Composé de formule I selon la revendication 1 ou 2, caractérisé en ce que $R_1$ représente un reste alcényle en $C_2$-$C_4$ mono-substitué par le chlore.

4. Composé de formule I selon la revendication 3, caracterisé en ce que $R_1$ représente le reste $Cl$-$CH=CH$-$CH_2$-.

5. Composé de formule I selon la revendication 1 ou 2, caractérisé en ce que $R_1$ représente le reste

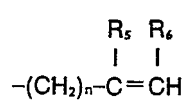

dans lequel $R_5$ et $R_6$ représentent chacun, indé-

pendamment l'un de l'autre, l'hydrogène ou le chlore, et n est égal à 0 ou 1.

6. Composé de formule I selon la revendication 1, 3, 4 ou 5, caractérisé en ce que $R_2$ et $R_3$ représentent l'hydrogène.

7. Composé de formule I selon les revendications 1 à 6, caractérisé en ce que $R_4$ représente le fluor.

8. Composé selon la revendication 7, de formule

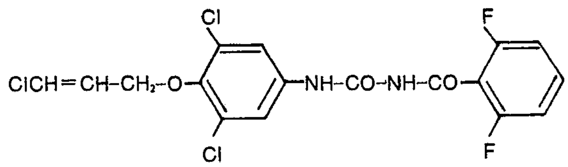

9. Composé selon la revendication 7, de formule

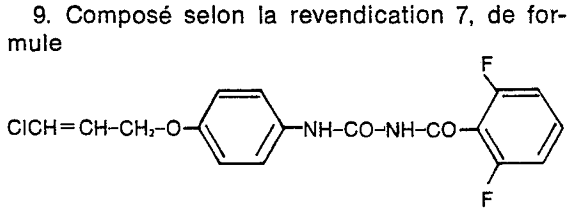

10. Composé selon la revendication 7, de formule

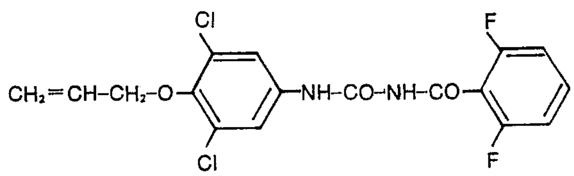

11. Composé selon la revendication 2, de formule

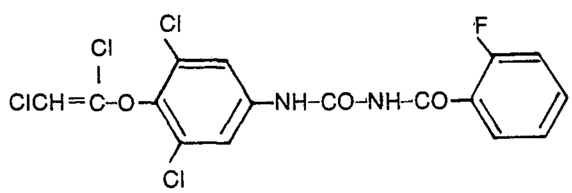

12. Composé selon la revendication 2, de formule

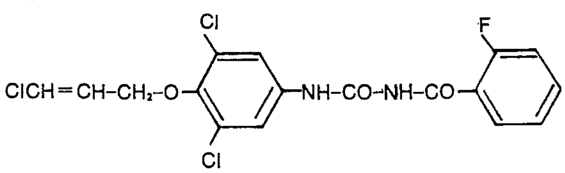

13. Procédé de préparation d'un composé selon les revendications 1 à 12, caractérisé en ce que

a) on fait réagir un composé de formule II

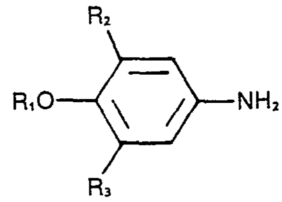

(II)

avec un composé de formule III

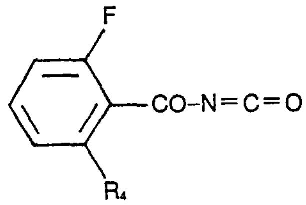

(III)

ou bien

b) on fait réagir un composé de formule IV

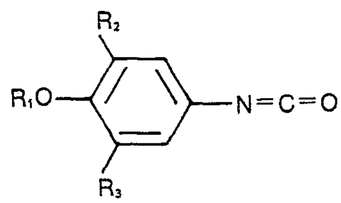

(IV)

avec un composé de formule V

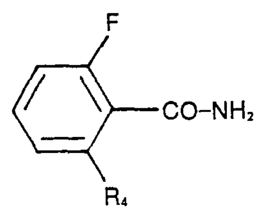

(V)

les symboles $R_1$, $R_2$, $R_3$ et $R_4$ des formules II à V ayant les significations indiquées dans les revendications 1 à 7.

14. Produits pesticides contenant en tant que composant actif un composé selon les revendications 1 à 12 avec des véhicules appropriés et/ou d'autres additifs.

15. Utilisation d'un composé selon les revendications 1 à 12 dans la lutte contre les parasites, de préférence les insectes et les représentants de l'ordre des acariens.